(19) **Europäisches Patentamt / European Patent Office / Office européen des brevets**

(11) **EP 1 935 326 B1**

(12) **EUROPEAN PATENT SPECIFICATION**

(45) Date of publication and mention
of the grant of the patent:
**30.11.2016 Bulletin 2016/48**

(21) Application number: **06811546.8**

(22) Date of filing: **04.10.2006**

(51) Int Cl.:
**A61B 1/00** *(2006.01)* **G01N 21/64** *(2006.01)*

(86) International application number:
**PCT/JP2006/320234**

(87) International publication number:
**WO 2007/043537 (19.04.2007 Gazette 2007/16)**

(54) **BIODIAGNOSTIC APPARATUS**

BIODIAGNOSTISCHES GERÄT

APPAREIL DE BIODIAGNOSTIC

(84) Designated Contracting States:
**DE FR GB**

(30) Priority: **12.10.2005 JP 2005297367**

(43) Date of publication of application:
**25.06.2008 Bulletin 2008/26**

(73) Proprietor: **Olympus Corporation**
**Tokyo 192-8507 (JP)**

(72) Inventor: **MACHIDA, Ryo**
**Hachioji-shi,**
**Tokyo 192-8507 (JP)**

(74) Representative: **von Hellfeld, Axel et al**
**Wuesthoff & Wuesthoff**
**Patentanwälte PartG mbB**
**Schweigerstrasse 2**
**81541 München (DE)**

(56) References cited:
**DE-A1- 19 548 913     DE-U1- 29 620 732**
**JP-A- 2003 207 451     JP-A- 2004 358 051**
**JP-A- 2006 020 727     JP-A- 2006 187 322**

**Description**

TECHNICAL ART

The present disclosure relates generally to a biodiagnosis apparatus, and more particularly to a biodiagnosis apparatus that lends itself to fluorescent observation and diagnosis of tumors or the like in a living body in general, and the bladder in particular.

BACKGROUND ART

For the observation of tumors, there has been a method known so far in the art, which makes use of a chemical compound that tends to gather at cancer cells and emits fluorescence upon irradiation with light (see Patent Publication 1). It is said there that not only a fluorescent image but also a direct image is simultaneously observable. Patent Publication 1 shows that the wavelength characteristics of an illumination system and an observation system overlap and are defined for the total transmittance of the whole system.

In that case, reflection images of sites other than a fluorescent site are observable through excitation light taken by the overlap of both characteristics slightly in the observation system.

Specifically, when 5-ALA (aminolevulinic acid) is used as the fluorescent chemical, the excitation light wavelength is $\lambda_e$=about 410 nm and the fluorescence wavelength is $\lambda_f$=630 nm. The florescence wavelength is red and, to observe this fluorescent site in good enough contrast, the background color should preferably be blue, and close to a monochrome. As the background color gets close to white, color contrasts grow unacceptably low.

Patent Publication 1 is JPH11511369 A.

Thus, the florescence wavelength is red and when reflection images of sites other than the fluorescent site are made observable through light excitation light taken in the observation system, there is the self-fluorescence of the living body to be observed among factors affecting the background color. As an optical system is designed without taking care of the self-fluorescence of the living body, it gives rise to a drop of color contrasts. The aforesaid prior art takes aim at making sure brightness contrasts: it says nothing about details of color contrasts.

SUMMARY OF THE DISCLOSURE

In view of such problems with the prior art as described above, the object of the disclosure is to provide a biodiagnosis apparatus in which the wavelength characteristics of an illumination system and an observation system overlap to thereby take excitation light in the observation system so that reflection images of sites other than a fluorescent site are observable, and which enables fluorescent observation to be implemented in good enough color contrast.

The invention is defined by the appended claim 1.

According to one aspect of the disclosure, there is provided a biodiagnosis apparatus harnessing fluorescent reactions of a living body tissue, which comprises a light source, a light source optical system, a light transfer system for guiding illumination light from said light source to the living body, an excitation light filter interposed between said light source and said light transfer system, an image transfer system for guiding light from the living body to an image plane, and an excitation cut filter located in said image transfer system, and in which spectral characteristics of illumination light transmitting through said excitation light filter and transmittance characteristics of said excitation cut filter have an overlapping portion, characterized in that:

upon observation of a subject under said biodiagnosis apparatus, spectral characteristics of a site of the image plane other than a fluorescent site satisfy the following condition (1):

$$0.005 \leqq I(\lambda_p + \Delta) / I(\lambda_p) \leqq 0.5 \qquad \cdots (1)$$

provided that $40 \leqq \Delta \leqq 80$nm, where $\lambda_p$ is a wavelength (nm) at which spectral intensity reaches a maximum, and $I(\lambda_p)$ is indicative of the then spectral intensity.

BRIEF DESCRIPTION OF THE DRAWINGS

Fig. 1 is an illustrative schematic sketch of the construction of the biodiagnosis apparatus according to the disclosure

Fig. 2 is an illustrative diagram of the transmittance characteristics of the excitation light filter (Fig.2a) and excitation cut filter (Fig.2b) in a prior art biodiagnosis apparatus.

Fig. 3 is an illustrative diagram of the system performance throughout the conventional biodiagnosis apparatus.

Fig. 4 is an indicative diagram of the values, as calculated and found, of the wavelength characteristics of the overlapping portion (background site) of the wavelength characteristics of the illumination system and the wavelength characteristics of the observation system in the event that there are the system performance of Fig. 3.

Fig. 5 is an illustrative schematic sketch of the color contrasts of the fluorescent and background sites in the event that 5-ALA is used as a fluorescent chemical: (a) is indicative of a red fluorescent site against a blue background, and (b) of a red fluorescent site against a white background.

Fig. 6 is a spectral intensity distribution for the background site at the upper limit, median and lower limit of the overlapping amount of both characteristics of the illumination and observation systems.

Fig. 7 comprises indicative diagrams of the system performance for obtaining spectral characteristics at the upper limit, median and lower limit of the spectral intensity distributions shown in Fig. 6, respectively.

Fig. 8 is a diagram wherein a spectral intensity distribution for the background site at the upper limit, median and lower limit of the spectral intensity distributions shown in Fig. 6, respectively, are represented on a chromaticity diagram.

Fig. 9 is an indicative diagram of the transmittance characteristics of a light source filter in another example of the biodiagnosis apparatus according to the disclosure.

Fig. 10 is an indicative diagram of the system performance throughout the biodiagnosis system of the construction of the apparatus of Fig. 9.

Fig. 11 is an indicative diagram of the transmittance characteristics in a modification to that of Fig. 9 wherein two light source filters are used.

Fig. 12 is an illustrative view of an exemplary filter switchover mechanism in the event that two light source filters are used as in the case of Fig. 11.

Fig. 13 is an indicative diagram of the transmittance characteristics of an antireflection coat and an excitation light filter in an optical system of the light source unit in the biodiagnosis apparatus of Fig. 1.

Fig. 14 is an indicative diagram of the transmittance characteristics of a prior art IR cut white light filter and an exclusive IR cut filter located in a TV camera head in the biodiagnosis apparatus of Fig. 15.

Fig. 15 is an illustrative schematic sketch of an arrangement wherein a TV camera system is connected to the biodiagnosis apparatus of the invention for the purpose of taking images.

BEST MODE FOR CARRYING OUT THE INVENTION

The biodiagnosis apparatus of the disclosure is now explained with reference to its principles and examples.

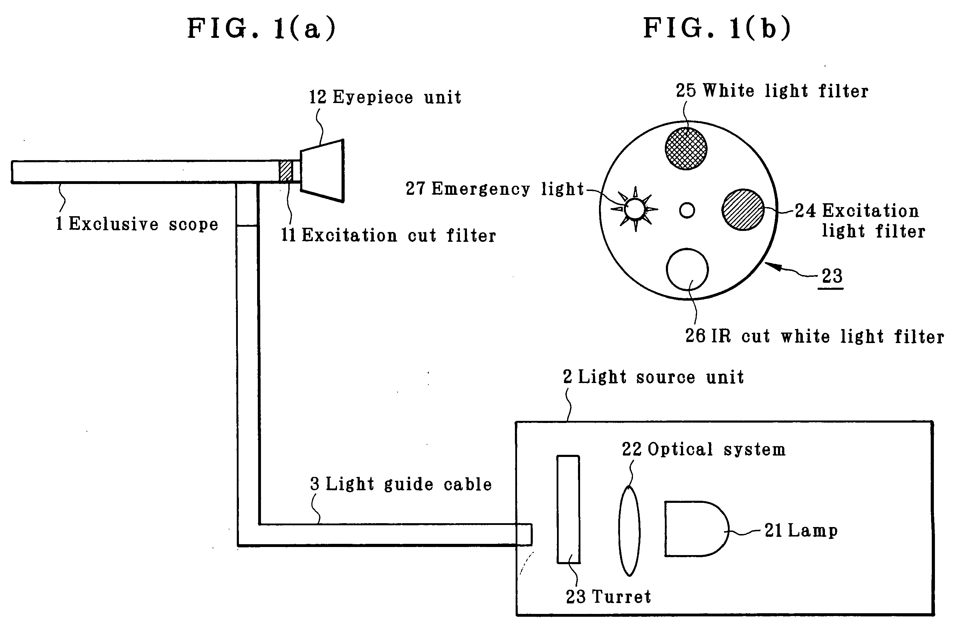

Fig. 1(a) is an illustrative schematic sketch of the construction of the biodiagnosis apparatus of the disclosure that comprises an exclusive scope (endoscope) 1 capable of being inserted into, for instance, the bladder and a light source unit 2 adapted to send illumination light to an illumination system for the exclusive scope 1 via a light guide cable 3. The light source unit 2 comprises a lamp 21 such as a xenon lamp, an optical system 22 adapted to condense light from the lamp 21 onto the input end of the light guide cable 3, and a turret 23 located in an optical path from the lamp 21. Around the turret 23, an excitation light filter 24, a white light filter 25, an IR cut white light filter 26 and an emergency light (small electric bulb) 27 are selectively disposed, as shown in Fig. 1(b). The excitation light filter 24 is one that has such wavelength characteristics as described later and excites fluorescent reactions; the white light filter 25 is one that transmits light all over the wavelength range to observe an ordinary image; the IR cut white light filter 26 is one that cuts off wavelengths in the infrared range to observe an image with good enough color reproducibility; and the emergency light (small electric bulb) 27 is to make sure the minimum illumination light for removing the exclusive scope 1 out of the living body when the lamp 21 goes off. An optical path through the exclusive scope 1 is provided in it with an excitation cut filter 11 so that an affected site can be directly observed via an eyepiece unit 12, or it can be observed via a CCD camera mounted to the eyepiece unit 12, as described later.

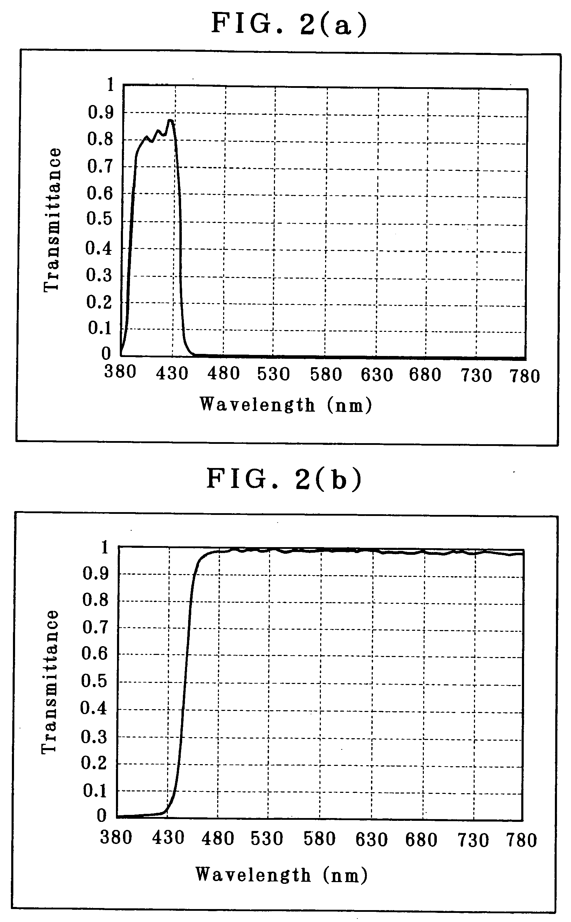

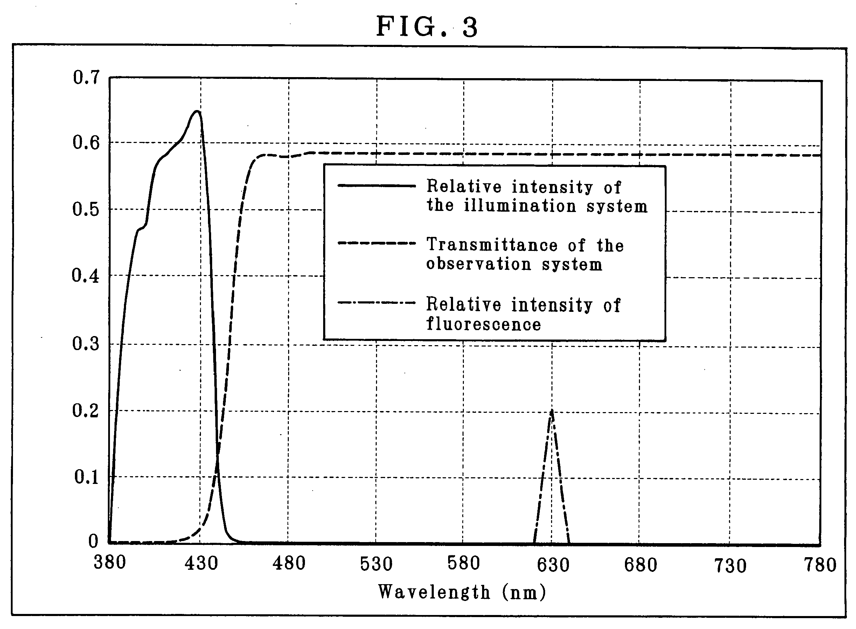

First, problems with a conventional biodiagnosis apparatus are explained. Fig. 2(a) is an indicative diagram of the transmittance characteristics of the excitation light filter 24, and Fig. 2(b) is an indicative diagram of the transmittance characteristics of the excitation cut filter 11. The excitation light filter 24 and excitation cut filter 11 have such transmittance characteristics; when the turret 23 is turned to insert the excitation light filter 24 in the illumination light path, the whole biodiagnosis system is going to have such system characteristics as shown in Fig. 3, wherein the wavelength characteristics (relative intensity) of an illumination system overlaps the wavelength transmittance of an observation system near a wavelength of 440 nm, and fluorescence of a fluorescent site excited by the excitation light of the illumination system is going to occur near a wavelength of 630 nm.

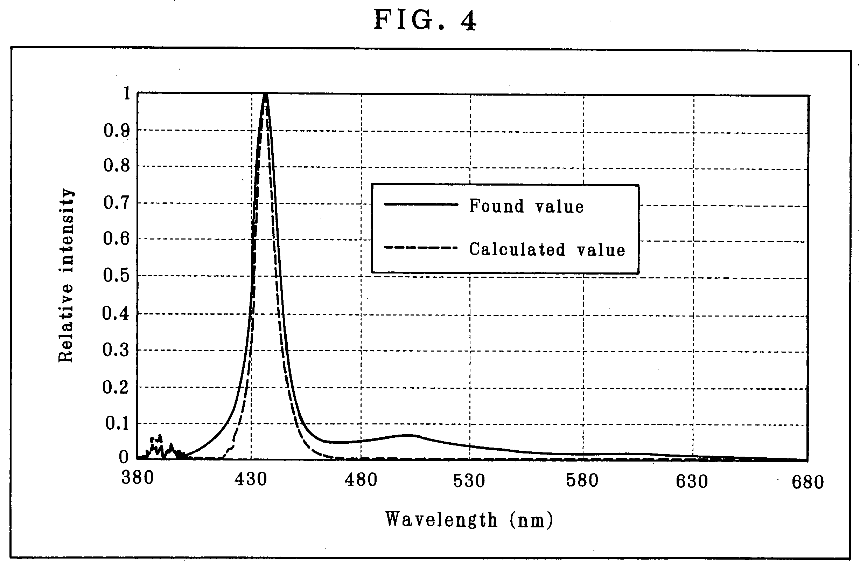

As regards the system characteristics of Fig. 3, Fig. 4 is an indicative diagram of the values of the wavelength characteristics, as calculated and found, of a background site (with none of fluorescent emission) that is defined by the overlap of the wavelength characteristics of the illumination system with that of the observation system. The calculated value here is the product of the wavelength characteristics of the illumination system and the wavelength transmittance of the observation system corresponding to the characteristics of Fig. 3, and the found value is greater than the calculated value especially in a wide range of wavelengths longer than the wavelength of 440 nm. This portion of the background site greater than the calculated value would appear to be caused by the self-fluorescence of the living body. For this reason, if there is a background color selected without taking care of this self-fluorescence of the living body, it will then give rise to a drop of color contrasts between the fluorescent site and the background site. This is the problem with the biodiagnosis apparatus set forth in Patent Publication 1.

As can be seen from the foregoing, the background color at the time of fluorescent observations is determined by an excitation light portion taken slightly in the observation system by the overlap of the wavelength characteristics of the illumination system and the observation system, and the self-fluorescence of the living body under observation as well.

Here, the excitation light directed on the subject (an affected site in the living body) stays constant, so is the quantity of self-fluorescence emanating out of the living body. The quantity of excitation light taken in the observation system is determined by the amount of overlap of both characteristics of the illumination system and the observation system: the ratio between that quantity of excitation light and the quantity of self-fluorescence is determined by the amount of overlap of both characteristics of the illumination system and the observation system. For this reason, the contrast of the background color, i.e., the color contrast between the fluorescent site and the background site is going to be determined by the amount of overlap of both characteristics of the illumination system and the observation system.

For observations in good enough contrasts, of importance is the ratio between the excitation light taken in the observation system and the self-fluorescence of the living body. As the amount of overlap grows large, the self-fluorescence of the living body becomes relatively smaller as compared with the excitation light taken in: the background color gets close to a monochrome. As the amount of overlap becomes small, on the contrary, the self-fluorescence of the living body grows large with respect to excitation light: the background color gets close to white.

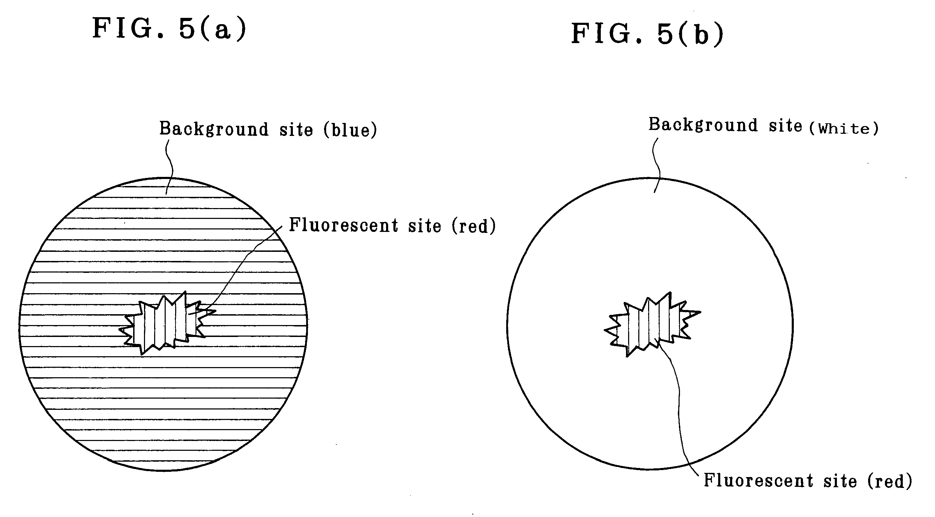

Fig. 5 is an illustrative schematic sketch of the color contrasts between the fluorescent site and the background site in the event that 5-ALA (aminolevulinic acid) is used as the fluorescent chemical. When there is a red fluorescent site against a blue background site, there is a good color contrast obtained so that the fluorescent site (affected site) is easy to see, as can be seen from Fig. 5(a). As there is a red fluorescent site against a white background as shown in Fig. 5(b), on the contrary, there is no good contrast obtained: the fluorescent site (affected site) is difficult to see, resulting in an increased probability of overlooking.

Therefore in the invention, the spectral characteristics of the background site other than the fluorescent site on the image plane upon observation of a subject should satisfy the following condition:

$$0.005 \leqq I(\lambda_p + \Delta)/I(\lambda_p) \leqq 0.5 \qquad \cdots (1)$$

provided that $40 \leqq \Delta \leqq 80$nm, where $\lambda_p$ is the wavelength (nm) at which the spectral intensity reaches a maximum, and $I(\lambda_p)$ is indicative of the spectral intensity at that wavelength $\lambda_p$.

As the upper limit of 0.5 to condition (1) is exceeded, there is an increase in the proportion of the self-fluorescence of the living body relative to the excitation light taken in by the overlap of characteristics. As a result, the background color gets close to white, giving rise to a drop of color contrasts, which may otherwise make it difficult to see the fluorescent site (affected site), resulting in an increased probability of overlooking.

As the lower limit of 0.005 to condition (1) is not reached, on the other hand, there is a decrease in the proportion of the self-fluorescence of the living body relative to the excitation light taken in by the overlap of characteristics, because a lot more excitation light is taken in the observation system. As a result, there is light reflected off sites other than the fluorescent site in addition to the fluorescence from the subject; that is, the background becomes all too bright, giving rise to a drop of brightness contrasts.

Especially when 5-ALA is used as the fluorescent chemical, the excitation light wavelength is $\lambda_e = 410$ nm and the fluorescence wavelength is $\lambda_f = 630$ nm. To observe orange or red fluorescence in good enough contrasts, the background color must tend to become a complementary color, or it should preferably be blue.

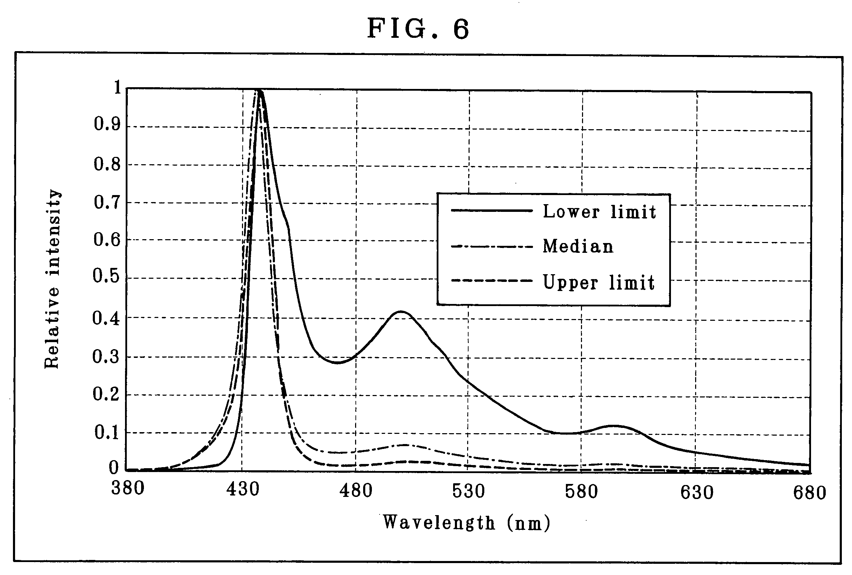

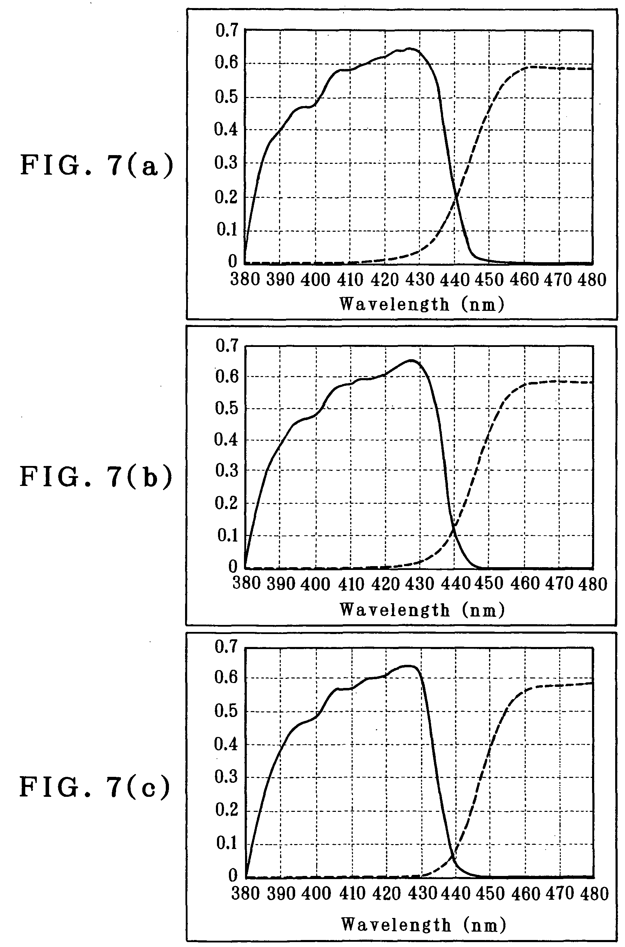

Fig. 6 is indicative of the spectral intensity distributions of the background site at the upper limit, median and lower limit (corresponding to the upper limit, median and lower limit of condition (1), respectively) of the amount of overlap of both characteristics of the illumination system and the observation system, and Fig. 7(a), 7(b) and 7(c) are indicative of the system performances for obtaining the spectral characteristics at the upper limit, median and lower limit of Fig. 6, respectively. In Fig. 7 here, note that each solid line is indicative of the wavelength characteristics (relative intensity) of the illumination system, and each broken line is indicative of the wavelength transmittance of the observation system.

The background at the lower limit of Fig. 6 (Fig. 7(c) is whitish blue, the background at the median (Fig. 7(b)) is blue, and the background at the upper limit (Fig. 7(a)) is deep blue.

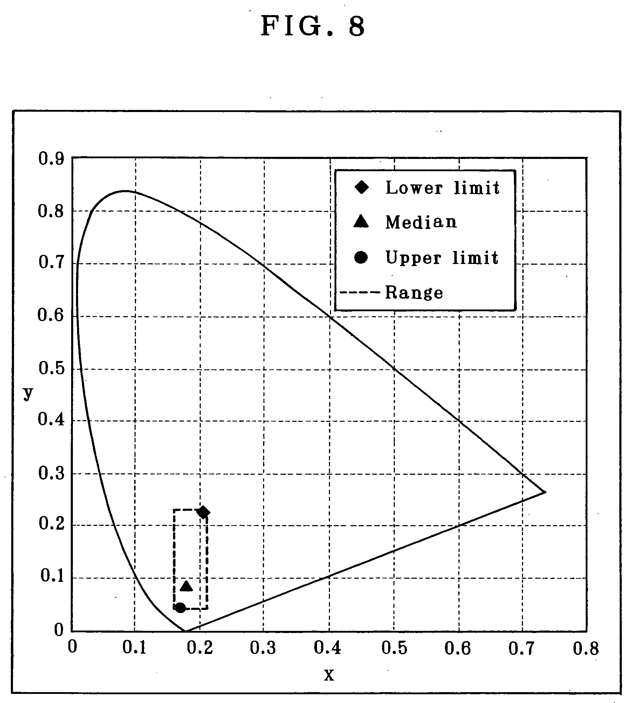

The spectral intensity distributions of the background site at the upper limit, median and lower limit (corresponding to the lower limit, median and upper limit of condition (1), respectively) of the amount of overlap of both characteristics of the illumination system and the observation system in Fig. 6 may also be expressed on a chromaticity diagram (CIE(X, Y)). On Fig. 8, chromaticity coordinates (x, y) at the aforesaid upper limit, median and lower limit are plotted. As can be appreciated from Fig. 8, it is desired that the chromaticity coordinates for light (on the image plane) after transmitting through the excitation cut filter 11 satisfies the following condition (2).

$$0.16 \leqq x \leqq 0.21, \quad 0.04 \leqq y \leqq 0.23 \qquad \cdots (2)$$

The range of condition (2) is marked off by a broken line in Fig. 8. Exceeding the upper limits of 0.21 and 0.23 to condition (2) causes the background color to get to white, giving rise to a drop of color contrasts between the fluorescent site and the background site. This in turn makes it difficult to view the fluorescent site (affected site), resulting in an increased probability of overlooking. Falling short of the lower limits of 0.16 and 0.04 to condition (2) is not desirable because there is a monochrome with an extremely noticeable blue tint. With visual sensitivity in mind, the background gets dark, giving rise to inconvenience that an image of light reflected off sites other than the fluorescent site is hardly viewable, even when the energy of the excitation light taken in the observation system is the same.

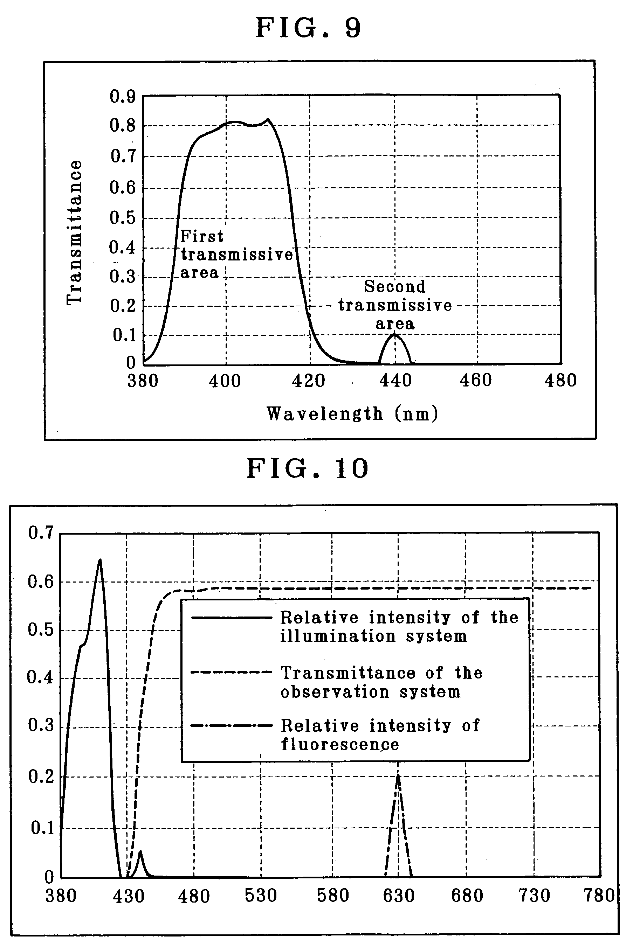

To obtain an image of light reflected off sites other than the fluorescent site in an image under observation, a light source filter (located instead of the excitation light filter 24) disposed in the illumination light path may be provided with a first transmissive area and a second transmissive area, with such transmittance characteristics as in Fig. 9. In this case, the first transmissive area is designed to contain an excitation wavelength for fluorescence (in the case of 5-ALA, the excitation light wavelength is $\lambda_e$=410 nm), and the second transmissive area is designed to be contained in the transmissive area of the excitation cut filter 11.

With the thus constructed arrangement, the whole biodiagnosis system has such system performances as shown in Fig. 10, wherein light transmitting through the second transmissive area is used to obtain a reflection image for the background other than the fluorescent site.

In this case, too, it is desired that the spectral characteristics of the background site other than the fluorescent site on the image plane upon observation of the subject satisfies the following condition (1):

$$0.005 \leqq I(\lambda_p + \Delta)/I(\lambda_p) \leqq 0.5 \qquad \cdots (1)$$

provided that $40 \leqq \Delta \leqq 80$nm, where $\lambda_p$ is the wavelength (nm) at which the spectral intensity reaches a maximum, and $I(\lambda_p)$ is indicative of the spectral intensity at that wavelength $\lambda_p$.

As the upper limit of 0.5 to condition (1) is exceeded, there is an increase in the proportion of the self-fluorescence of the living body relative to the light passing through the second transmissive area. As a result, the background color gets close to white, giving rise to a drop of color contrasts, which may otherwise make it difficult to see the fluorescent site (affected site), resulting in an increased probability of overlooking.

As the lower limit of 0.005 to condition (1) is not reached, on the other hand, there is a decrease in the proportion of the self-fluorescence of the living body relative to the light transmitting through the second transmissive area, because the light transmitting through the second transmissive area enters more the observation system. As a result, there is light reflected off sites other than the fluorescent site in addition to the fluorescence from the subject; that is, the background becomes all too bright, giving rise to a drop of brightness contrasts.

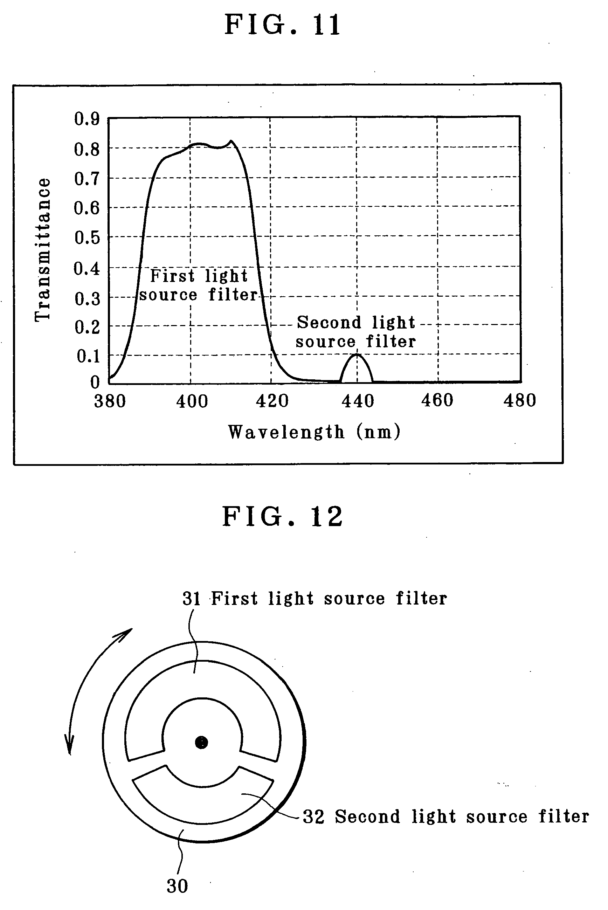

It is noted that instead of providing the light source filter disposed in the illumination light path with the first and the second transmissive area, two light source filters may be used. The first light source filter with such transmittance characteristics as shown in Fig. 11 is designed to contain the excitation wavelength for fluorescence as is the case with the aforesaid first transmissitve area, and the second light source filter is designed to be contained in the transmissive area of the excitation cut filter 11 as is the case with the aforesaid second transmissive area.

Then, the light transmitting through the first light source filter and the light transmitting through the second light source filter are sequentially directed to the subject so that by making use of the afterimage phenomenon of the observer's eye or field sequential video image pickup, the fluorescent and background sites can be superposed to implement observation in good enough color contrasts. To direct sequentially to the subject the light transmitting through the first light source filter and the light transmitting through the second light source filter, such a turret 30 as shown in Fig. 12 is located. Around that turret 30 a first light source filter 31 and a second light source filter 32 are disposed in a continuously switchover way to turn that turret 30 continuously or reciprocally.

As a light source for the illumination light directed to the subject, a laser that oscillates an excitation light wavelength may be used instead of the light source filter having such transmittance characteristics as shown in Fig. 9. Then, light of a wavelength contained in the transmissive area of the excitation cut filter 11 is superposed on the excitation light coming from that laser, or directed sequentially to the subject in an alternate manner.

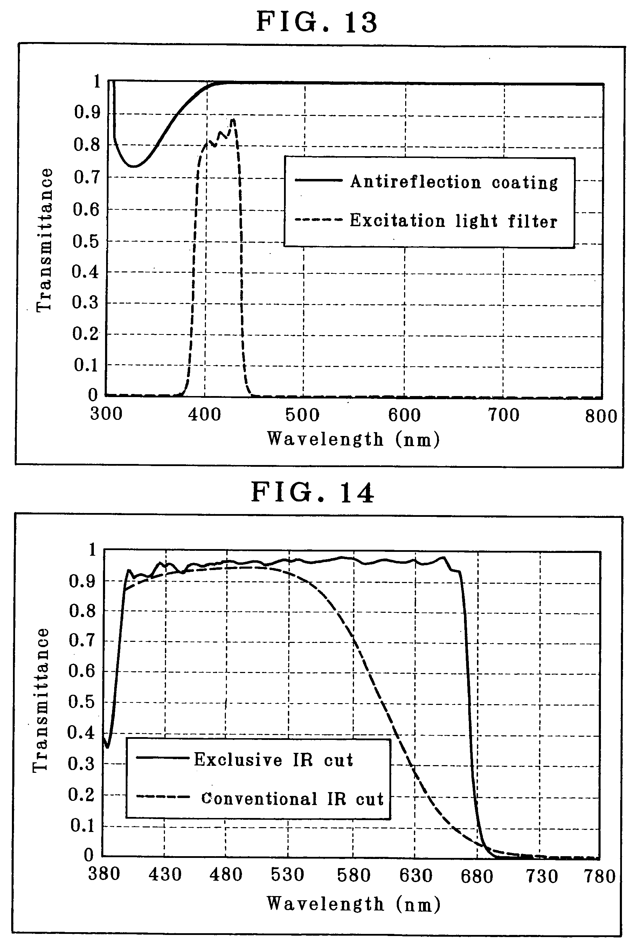

Referring back to the biodiagnosis apparatus of Fig. 1(a), the optical system 22 must not attenuate excitation light transmitting through the excitation light filter 24; as can be seen from the wavelength characteristics of Fig. 13, it is of importance that an antireflection coating applied onto a lens, etc. in the optical system 22 should have high transmittance with respect to the wavelength transmitting through the excitation light filter 24.

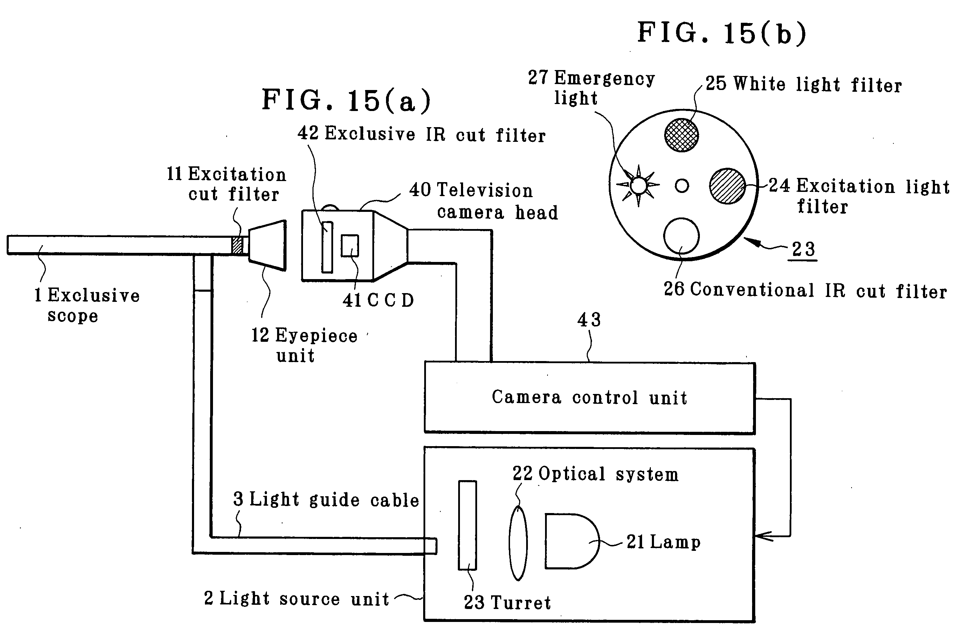

Fig. 15(a) is illustrative in schematic of an arrangement wherein a TV camera system is connected to the biodiagnosis apparatus of Fig. 1(a) for imaging purposes. As is the case with Fig. 1, the biodiagnosis apparatus itself comprises an exclusive scope (endoscope) 1 capable of being inserted into, for instance, the bladder, and a light source unit 2 for sending illumination light to that exclusive scope 1 via a light guide cable 3. The light source unit 2 comprises a lamp 21 such as a xenon lamp, an optical system 22 adapted to condense light from the lamp 21 onto the input end of the light guide cable 3, and a turret 23 located in an optical path from the lamp 21. Around the turret 23, an excitation light filter 24, a white light filter 25, a conventional IR cut white light filter 26 and an emergency light (small electric bulb) 27 are selectively disposed, as shown in Fig. 15(b). The excitation light filter 24 is one that has such wavelength characteristics as described above and transmits light containing an excitation wavelength; the white light filter 25 is one that transmits light all over the wavelength range to observe an ordinary image; the conventional IR cut white light filter 26 is one that cuts off wavelengths in the infrared range and transmits light in the visible range to observe an image with good color reproducibility, as can be seen from the transmittance characteristics of Fig. 14 for conventional IR cutting; and the emergency light (small electric bulb) 27 is to make sure the minimum illumination light for pulling the exclusive scope 1 out of the living body when the lamp 21 goes off. An optical path through the exclusive scope 1 is provided in it with an excitation cut filter 11 and an eyepiece unit 12 for the observation of an affected site.

And, in the exemplary arrangement here, a TV camera head 40 is mounted on the eyepiece unit 12 of the biodiagnosis apparatus per se to enable a subject to be electronically imaged. The TV camera head 40 has in it a CCD 41 and an exclusive IR cut filter 42 located on the entrance side of CCD 41. That exclusive IR cut filter 42 is used instead of an absorption type IR cut filter such as the conventional IR cut filter of Fig. 14 for the purpose of keeping ordinary color reproducibility as conventional. To this end, a filter with a cutoff frequency shifted to a longer wavelength side is used in such a way as to have no influence on a fluorescent image (fluorescent wavelength $\lambda_f$=630 nm).

Video signals obtained from the TV camera head 40 are forwarded to a camera control unit 43 to display and record taken images, and feedback signals are forwarded from the camera control unit 43 to the light source unit 2 for light control, etc.

INDUSTRIAL APPLICABILITY

The disclosure provides a biodiagnosis apparatus
wherein the wavelength characteristics of an illumination system and an observation system overlap so that a reflection image other than at a fluorescent site can be observed through excitation light taken by that overlap in the observation system. The disclosed apparatus is designed to
satisfy condition (1) or (2) so that the fluorescent site can be observed in good enough color contrasts against the background.

**Claims**

1.  Method of selecting the viewing condition of an imaging plane of a bladder viewed using the pre-inserted biodiagnosis apparatus and harnessing the fluorescent reactions of the living body, said biodiagnosis apparatus comprising a light source unit (2) comprising a light source (21), a light source optical system (22), a light transfer system (3) adapted to guide illumination light from said light source unit (2) to the living body, an excitation light filter (24) interposed between said light source unit (2) and said light transfer system (3), an image transfer system (1, 12) adapted to guide light from the living body to an image plane, and an excitation cut filter (11) located in said image transfer system (1, 12), and in which apparatus spectral transmittance characteristics of said excitation light filter (24) and a transmittance characteristic of said excitation cut filter (11) have an overlapping portion, and wherein the light source unit (2) comprises a turret (23) located in an optical path from the light source (21) and wherein said excitation light filter (24), a white light filter (25) and an emergency light (27) are selectively disposed around the turret (23), wherein by selection of the excitation cut filter, the viewing condition is attained that, upon observation of said bladder, chromaticity coordinates (x, y) for
light on the image plane for said illumination light after transmitting through said excitation cut filter (24) satisfies the following condition:

$$0.16 \le x \le 0.21, \ 0.04 \le y \le 0.23.$$

**2.** The method of claim 1 in which the biodiagnosis apparatus turret (23) comprises a light source filter comprising a first transmissive area (31) containing a fluorescence excitation wavelength and a second transmissive area (32) acting as the excitation cut filter, wherein a transmittance characteristics of said excitation cut filter (11) and that of said second transmissive area (32) of said light source filter (30) have the overlapping portion.

**3.** The method of claim 2, wherein the biodiagnosis apparatus turret comprises a first light source filter in alignment with said first transmissive area (31) and a second light source filter in alignment with said second transmissive area (32), and wherein the light source directs sequentially to the subject light transmitting through said first light source filter and light transmitting through said second light source filter.

## Patentansprüche

**1.** Verfahren zum Auswählen der Sehbedingungen einer Bildebene einer Blase, die betrachtet wird durch Benutzen eines vorher eingeführten biodiagnostischen Geräts und Nutzen der fluoreszenten Reaktionen des lebendigen Körpers, wobei das biodiagnostische Gerät eine eine Lichtquelle (21) umfassende Lichtquelleneinheit (2), ein Lichtquellen-Optik-System (22), ein Lichtübertragungssystem (3) ausgebildet zum Lenken eines Beleuchtungslichts von der Lichtquelleneinheit (2) zu dem lebenden Körper, einen Anregungslichtfilter (24) angeordnet zwischen der Lichtquelleneinheit (2) und dem Lichtübertragungssystem (3), ein Bildübertragungssystem (1, 12) ausgebildet zum Lenken von Licht von dem lebendigen Körper zu einer Bildebene, und einen Anregungslichtschnittfilter (11) angeordnet in dem Bildübertragungssystem (1, 12) umfasst, und in welchem Gerätspektralübertragungseigenschaften des Anregungslichtfilters (24) und eine Übertragungseigenschaft des Anregungslichtschnittfilters (11) einen überlappenden Bereich aufweisen, und wobei die Lichtquelle (2) einen in einem optischen Pfad von der Lichtquelle (21) angeordneten Revolver (23) umfasst, und wobei der Anregungslichtfilter (24), ein Weißlichtfilter (25) und ein Notfalllicht (27) auswählbar in dem Revolver (23) angeordnet sind, wobei durch Auswählen des Anregungsschnittfilters die Sehbedingung erreicht wird, dass, auf eine Betrachtung der Blase hin, Chromatizitätskoordinaten (x, y) für Licht auf der Bildebene für das Beleuchtungslicht nach dem Übertragen durch den Anregungsschnittfilter (24) die folgende Bedingung erfüllt:

$$0{,}16 \ \le x \le 0{,}21, \ 0{,}04 \le y \le 0{,}23.$$

**2.** Verfahren nach Anspruch 1, in welchem der biodiagnostische Gerätsrevolver (23) einen Lichtquellenfilter umfasst, der einen eine Fluoreszenzanregungswellenlänge beinhaltenden ersten durchlässigen Bereich (31) und einen zweiten durchlässigen Bereich (32), der als der Anregungslichtschnittfilter agiert, umfasst, wobei eine Übertragungseigenschaft des Anregungslichtschnittfilters (11) und die des zweiten durchlässigen Bereichs (32) des Lichtquellenfilters (30) einen überlappenden Bereich aufweisen.

**3.** Verfahren nach Anspruch 2, wobei der biodiagnostische Gerätsrevolver einen ersten Lichtquellenfilter in einer Richtung mit dem ersten durchlässigen Bereich (31) und einen zweiten Lichtfilter in einer Richtung mit dem zweiten durchlässigen Bereich (32) umfasst, und wobei die Lichtquelle aufeinanderfolgend zu dem Subjekt Licht, das sich durch den ersten Lichtquellenfilter überträgt, und Licht, das sich durch den zweiten Lichtquellenfilter überträgt, richtet.

## Revendications

**1.** Procédé de sélection de la condition de visualisation d'un plan d'imagerie d'une vessie visualisée en utilisant un appareil de biodiagnostic pré-inséré et exploitant les réactions de fluorescence du corps vivant, ledit appareil de biodiagnostic comprenant une unité (2) de source de lumière comprenant une source de lumière (21), un système optique (22) à source de lumière, un système (3) de transfert de lumière adapté pour guider une lumière d'éclairage depuis ladite unité (2) de source de lumière vers le corps vivant, un filtre (24) de lumière d'excitation interposé entre ladite unité (2) de source de lumière et ledit système (3) de transfert de lumière, et un système (1, 12) de transfert d'image adapté pour guider la lumière du corps vivant vers un plan d'image, et un filtre (11) de coupure d'excitation placé dans ledit système (1, 12) de transfert d'image, et dans lequel appareil des caractéristiques de transmittance spectrale dudit filtre (24) de lumière d'excitation et une caractéristique de transmittance dudit filtre (11) de coupure d'excitation possèdent une partie de chevauchement, et dans lequel l'unité (2) de source de lumière comprend une tourelle (23) placée dans un trajet optique partant de la source de lumière (21) et dans lequel ledit filtre (24) de lumière d'excitation, un filtre (25) de lumière blanche et une lumière de secours (27) sont sélectivement disposés autour de la tourelle (23), dans lequel, par la sélection du filtre de coupure d'excitation, la condition de visualisation est atteinte qui réside en ce que, sur observation de ladite vessie, les coordonnées de chromaticité (X, Y) de la lumière sur le plan d'image pour ladite lumière d'éclairage après transmission à travers ledit filtre (24) de coupure d'excitation satisfont la condition suivante :

$$0,16 \leq x \leq 0,21,\ 0,04 \leq y \leq 0,23.$$

2. Procédé selon la revendication 1, dans lequel la tourelle (23) de l'appareil de biodiagnostic comprend un filtre de source de lumière comprenant une première zone de transmission (31) contenant une longueur d'onde d'excitation de fluorescence et une deuxième zone de transmission (32) agissant en tant que filtre de coupure d'excitation, dans lequel une caractéristique de transmittance dudit filtre (11) de coupure d'excitation et celle de ladite deuxième zone de transmission (32) dudit filtre (30) de source de lumière possèdent la partie de chevauchement.

3. Procédé selon la revendication 2, dans lequel la tourelle de l'appareil de biodiagnostic comprend un premier filtre de source de lumière en alignement avec ladite première zone de transmission (31) et un deuxième filtre de source de lumière en alignement avec ladite deuxième zone de transmission (32), et dans lequel la source de lumière dirige séquentiellement vers le sujet la lumière traversant ledit premier filtre de source de lumière et la lumière traversant ledit deuxième filtre de source de lumière.

# FIG. 1(a)

12 Eyepiece unit

1 Exclusive scope

11 Excitation cut filter

3 Light guide cable

2 Light source unit

22 Optical system

3 Light guide cable

21 Lamp

23 Turret

# FIG. 1(b)

25 White light filter

27 Emergency light

24 Excitation
light filter

23

26 IR cut white light filter

EP 1 935 326 B1

## FIG. 2(a)

## FIG. 2(b)

FIG. 3

# FIG. 4

FIG. 5(a)

FIG. 5(b)

Background site (blue)

Fluorescent site (red)

Background site (White)

Fluorescent site (red)

EP 1 935 326 B1

# FIG. 6

FIG. 7(a)

FIG. 7(b)

FIG. 7(c)

# FIG. 8

## FIG. 9

## FIG. 10

# FIG. 11

# FIG. 12

31 First light source filter

32 Second light source filter

30

# FIG. 13

# FIG. 14

# FIG. 15(b)

## FIG. 15(a)

27 Emergency light

25 White light filter

42 Exclusive IR cut filter

11 Excitation cut filter

40 Television camera head

24 Excitation light filter

1 Exclusive scope

23

41 CCD

26 Conventional IR cut filter

12 Eyepiece unit

43

Camera control unit

22 Optical system

3 Light guide cable

21 Lamp

2 Light source unit

23 Turret

EP 1 935 326 B1

**EP 1 935 326 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H11511369 A